# EUROPEAN PATENT SPECIFICATION

(11) **EP 1 793 225 B1**
(45) Date of publication and mention of the grant of the patent: **29.05.2013**
(21) Application number: 05768473.0
(22) Date of filing: 04.08.2005
(51) Int. Cl.: G01N 29/06

(54) **Internal tree nondestructive inspection method and apparatus using acoustic tomography**
Zerstörungsfreies Verfahren und zerstörungsfreie Vorrichtung zur internen Inspektion von Bäumen mittels akustischer Tomographie
Méthode et système non-destructifs d"inspection de l'intérieur d"un arbre par tomographie acoustique

(30) Priority: 11.08.2004 JP 2004234842
(43) Date of publication of application: 06.06.2007
(73) Proprietor: JFE Civil Engineering, Tokyo 1110051 (JP); Incorporated Administrative Agency National Agriculture and Food Research Organization, Ibaraki (JP); Yamamoto Engineering Corporation, Miami, FL 33178 (US)
(72) Inventor: MOHRI, Yoshiyuki, Tsukuba-shi, Ibaraki 3058609 (JP); SAKAKIBARA, Junichi c/o JFE Civil Engineering & Construction Corp., Tokyo 1110051 (JP); YAMAMOTO, Tokuo, 33176 (US); TANAKA, Masato c/o JFE Civil Engineering & Construction Corp., Tokyo 1110051 (JP)
(74) Representative: Grünecker, Kinkeldey, Stockmair & Schwanhäusser
(86) International application number: PCT/JP2005/014308
(87) International publication number: WO 2006/016520

(56) References cited:
- JP-A- 2 098 663
- JP-A- 2 251 750
- JP-A- 10 068 779
- JP-A- 10 318 992
- JP-A- 60 039 556
- JP-A- 62 044 660
- JP-A- 2000 035 483
- US-B1- 6 347 551
- MARTINIS ROBERTO ET AL: "Ultrasonic tomography on standing trees", ANNALS OF FOREST SCIENCE, vol. 61, no. 2, March 2004 (2004-03), pages 157-162, XP002626480, ISSN: 1286-4560
- KIM W K ET AL: "Signal-to-noise ratio and bandwidth for pseudo-random codes in an ultrasonic imaging system", ULTRASONIC IMAGING, DYNAMEDIA INC., SILVER SPRING, MD, US, vol. 6, no. 3, 1 July 1984 (1984-07-01), pages 313-323, XP023046468, ISSN: 0161-7346, DOI: DOI:10.1016/0161-7346(84)90016-6 [retrieved on 1984-07-01]

## Description

### [Technical Field]

The present invention relates to internal tree nondestructive inspection method and apparatus, more specifically, to an internal tree nondestructive inspection method and apparatus using acoustic tomography capable of diagnosing hollows and decay inside a roadside tree or an important tree while it remains standing.

### [Background Art]

Prior art document US 6 347 551 B1 relates to a non-destructive analysis of standing and felled trees and poles by using an ultrasonic computed tomography. The non-destructive analysis comprises the steps of engaging a belt which is equipped with a plurality of transceivers arranged at a certain distance around a circumference of the tree. Thereby the transceivers, which can receive and transmit ultrasonic impulses, are provided at a plurality of positions at the circumference of the tree. In a next step one transceiver transmits anultrasonic impulse directed to the inside of the tree. The remaining transceivers receive said ultrasonic impulse and transmit the measured data to an infrared data transmitter provided in the belt. Said infrared data transmitter transmits the data from all receiving transceivers to a visualizing device like a laptop. Said steps are repeated several times changing the transceiver being the ultrasonic source in clockwise direction.

Prior art publication from Martinis Roberto et al: "Ultrasonic tomography on standing threes", discloses a non-destructive analysis for standing trees by using ultrasonic tomography. Said method comprises a step of generating an ultrasonic impulse with a source which is positioned during the measurement at 16 different positions at the circumference of the tree. At a first position, the ultrasonic impulse generated by the source penetrates the inside of the tree and is received by one receiver which is consecutively positioned on each of the remaining 15 positions. The data transmitted by the receiver is processed and displayed by a computer. Said procedure is repeated until the source has transmitted ultrasonic impulses on each of the 16 positions.

To evaluate the health of trees by investigating hollows and decay inside trees is very important to prevent damage to neighborhoods due to toppling of trees, consider the process of products of trees when felling trees to be sawn, and protect natural treasures such as Yakusugi and commemorative trees. However, it is difficult to grasp the internal state of a tree only by tree diagnosis from its external appearance, so that the inside of the tree must be diagnosed.

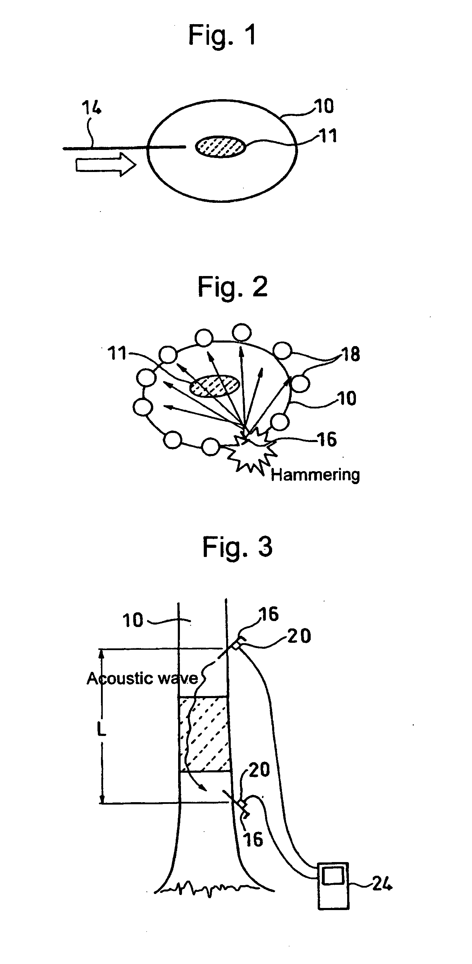

As a method for inspecting the inside of a standing tree in a destructive manner, as shown in Fig. 1, there is (1) a Resistograph in which a penetration resistance when drilling a tree 10 by using a drill or needle 14 is measured. In Fig. 1, the reference numeral 11 denotes a hollow or decay.

In addition, as a method for inspecting the inside of a standing tree in a nondestructive manner without damaging the tree, there are (2) a hammering sound inspection method in which a hammering when a nail 16 is driven with a hammer is measured by an accelerometer as shown in Fig. 2, (3) an acoustic wave inspection method in which a pulsed acoustic wave of several kHz or less is propagated in the vertical direction by nails 16 disposed at a distance L from each other at the upper and lower sides of the standing tree 10 and detected by sensors 20 and the strength and hardness of the tree are evaluated by an oscilloscope 24 as shown in Fig. 3, and (4) electrical detection in which differences in composition and density of containedmoisture are estimated from the natural potential, electrical resistance, and capacitance to grasp the state of decay of the tree.

As a diagnosis technique using acoustic tomography, there are techniques described in Japanese Published Unexamined Patent Applications No. H10-186048 and No. 2000-35483 proposed by a part of the applicant although these are not for diagnosis of trees.

However, in the Resistograph of (1), not only is the tree damaged but also the measuring is only possible up to the length of penetration of the needle 14, so that the tree size which can be measured by this method is limited. The metal needle 14 directly penetrates the tree 10, so that to prevent the tree from being damaged, the number of penetrations is limited to be several, however, unless the needle 14 hits a hollow or decay, the hollow or decay cannot be detected. Furthermore, there isdangerous risk with the work of penetration of the needle 14 and the work requires skill. The needle 14 is easily broken, and the expenses for repair mount, and these are problem points.

On the other hand, the nondestructive inspection methods of (2) through (4) do not damage trees, however, the hammering sound inspection method of (2) cannot control an frequency and amplitude of the sound, so that it is low in repeatability and has difficulty in reading of obtained signals. In addition, this method requires skill in reading of signals and has problems in objectivity and accuracy.

The acoustic wave inspection method of (3) needs a large-scale apparatus, and is low in accuracy and is incapable of detecting hollows.

In the electrical detection of (4), the results of measurement are greatly influenced by an amount of sap, etc., and greatly reflect a physiological state of a tree.

Use of an X-ray, gamma ray, or radar instead of the acoustic wave is also possible, however, apparatuses thereof have problems in handling performance and portability, and convenient measurements are difficult.

### [Disclosure of the Invention]

The present invention was made to solve the conventional problems described above, and an object thereof is to realize efficient diagnosis of the internal state of a tree at the site without damaging the tree.
The aforesaid problems are solved by an internal tree nondestructive inspection method according to claim 1 and an internal tree nondestructive inspection apparatus according to claim 8. Preferred embodiments are specified in the dependent claims.

According to the invention, an internal state of a standing tree can be efficiently inspected in a nondestructive manner without damaging the tree.

Therefore, (1) it becomes possible to prevent roadside trees from being toppled over and efficiently plant roadside trees. Namely, by introducing a convenient measuring method which does not rely on the skill of a craftsman, tree diagnosis can be quickly and accurately made at low cost. By accurately grasping hollows which will cause a tree to stand dead or toppling of a roadside tree, the level of toppling risk can be judged. In addition, by properly felling or displacing a tree with a high risk of toppling, the neighborhood of the tree can be prevented from being damaged by a fallen tree. By grasping and applying an efficient measure according to the level of risk, excessive felling is prevented and the cost of planting work can be remarkably reduced.

In addition, (2) it becomes possible to conserve important trees. Namely, in conservation of important trees such as natural treasures, diagnosis for taking proper measures can be made. Particularly, even in a case of a big tree with a diameter exceeding 1 meter whose internal diagnosis is very difficult, an internal state thereof including hollows and decay can be grasped, so that this is very effective for conservation of these important trees. In addition, among important trees, sacred trees or trees planted by Imperial family members which need perfectly nondestructive investigation cannot be substantially investigated by the existing techniques, however, by using the nondestructive investigation method of the present invention, an internal state of such a tree can be diagnosed.

### [BRIEF DESCRIPTION OF THE DRAWINGS]

Fig. 1 is a sectional view showing a conventional Resistograph using a penetration resistance;
Fig. 2 is a sectional view showing a conventional hammering sound inspection method;
Fig. 3 is a front view showing a conventional acoustic wave inspection method;
Fig. 4 (A) is a perspective view, Fig. 4 (B) is a block diagram, and Fig. 4(C) is a sectional view, showing a first embodiment of the invention;
Fig. 5 is a flowchart showing measurement steps of the invention;
Fig. 6 is a sectional view showing an example of a sensor attaching method of the invention;
Figs. 7 are sectional views showing a sensor attaching state of the invention;
Fig. 8 is a sectional view showing another example of a sensor attaching method of the invention;
Fig. 9 is a plan view showing a general drawing method of the invention;
Fig. 10 is a diagram showing a drawing example according to the method of Fig. 9;
Fig. 11 is a plan view showing a drawing method in the embodiment;
Figs. 12 are diagrams showing drawing examples in the embodiment;
Figs. 13 are diagrams showing effects of the invention;
Figs. 14 is a perspective view showing a construction of a second embodiment of the invention;
Fig. 15 is a drawing showing a variation of a sensor.

### [Best Mode for Carrying Out the Invention]

Hereinafter, embodiments of the invention will be described in detail with reference to the accompanying drawings.

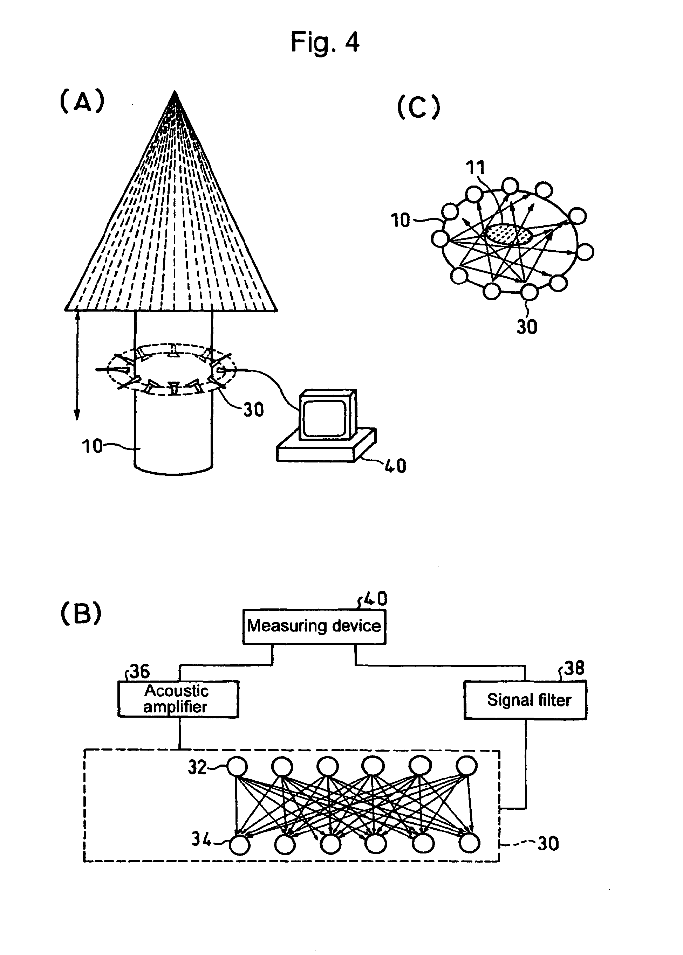

A first embodiment of the invention includes, as shown in Fig. 4, a number (for example, 8 or 16) of sensors 30 which commonly serve as oscillators 32 and small microphone 34 for making acoustic waves incident on the inside of a tree 10 and receiving acoustic waves propagated inside the tree; an acoustic amplifier 36 for amplifying acoustic waves made incident on the oscillators 32; a signal filter 38 for filtering predetermined components in signals received by the small microphones 34; and a measuring device (PC) 40 which inputs vibration signals into sensors which serve as the oscillators 32 among the sensors 30 and visualize an internal state of the tree based on outputs from sensors that serve as the small microphones 34 among the sensors 30.

The oscillators 32 and the small microphones 34 are formed to be common by using, for example, piezoelectric devices so that oscillating and receiving positions can be changed without changing the attachment positions thereof to the tree 10.

The acoustic wave to be made incident from the oscillator 32 is not a pulsedwave conventionallyusedbut, for example, acontinuous wave obtained by converting a sine wave by the PRBS codes and correlation of an oscillating wave and a received wave is determined to grasp the acoustic propagation as described in Japanese Published Unexamined Patent Applications No. H10-186048. Therefore, a measuring distance for even a high-frequency wave which cannot be normally propagated can be made long. Namely, in the conventional acoustic wave inspection method using a pulsed wave, (1) a wave transducer must be changed to change the frequency, (2) the signal is attenuated, so that the output must be made high, and (3) the output control cannot be performed, however, according to the invention, measurement becomes possible by using a continuous wave using PRBS codes, (1) oscillation can be generated while controlling the frequency, (2) measurement with high accuracy can be made at a low output (S/N ratio: about 1000 times), and (3) oscillation can be generated while making the output constant, so that evaluation using an acoustic pressure can be performed.

Particularly, when a piezoelectric device is used as the sensor 30, not only can it be used for both oscillating and receiving, but it is also small in size and can oscillate a PRBS-converted signal. Furthermore, by using the sensor 30 small in size, a branch at a high position can be measured.

In addition, by performing measurement while moving the sensors 30 vertically, measurement can be made at an arbitrary height.

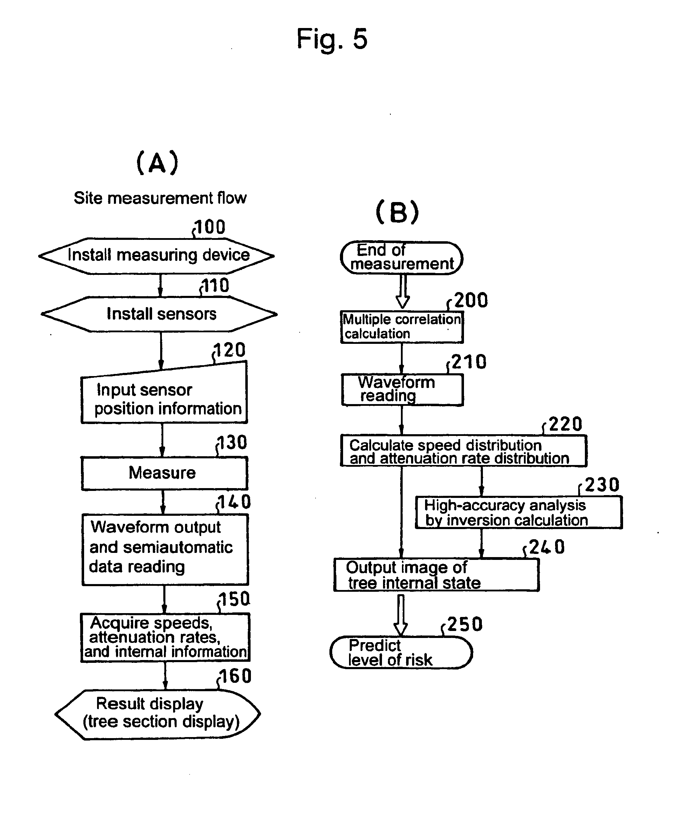

Hereinafter, measurement steps will be described with reference to Fig. 5.

First, at the site, a measuring device 40 is installed near the tree 10 at Step 100.

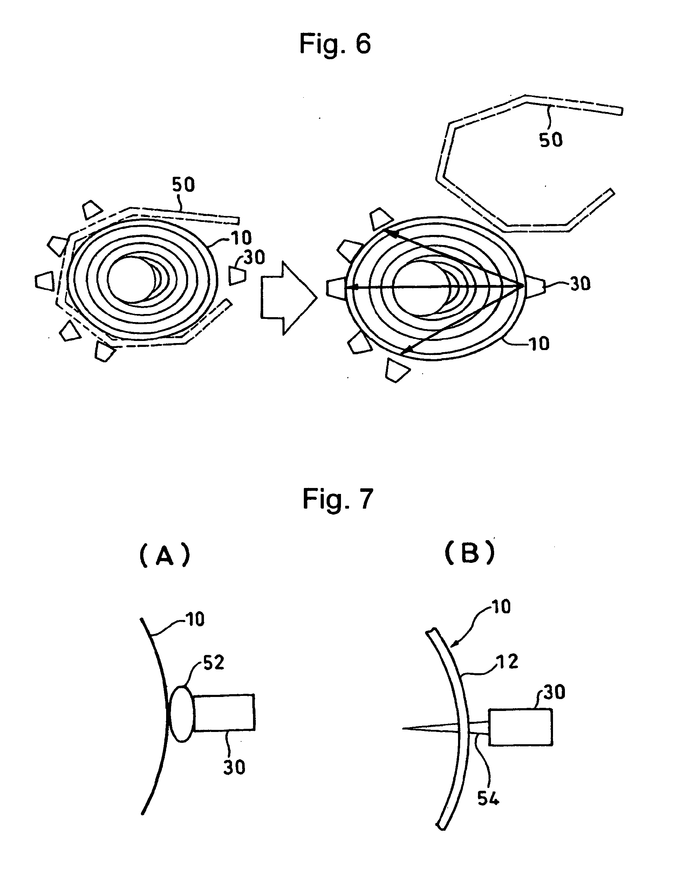

Then, at Step 110, the sensors 30 are set on the tree 10. The sensors can be directly attached to the tree 10 by clay 52 as shown in Fig. 7(A) or attached by being bonded to a nail 54 as shown in Fig. 7(B) by using, for example, a wire 50 as a guide as shown in Fig. 6. The clay 52 easily propagates sounds, and can fix the sensors 30 by its adhesive force. When the clay 52 is used, the tree 10 is not flawed. On the other hand, the nail 54 is preferable when the tree bark 12 is thick or a gap is present on the back of the tree bark 12. By using the clay 52 or nail 54, arbitrary layout becomes possible without depending on the shape of the tree, and accurate amplitude control becomes possible.

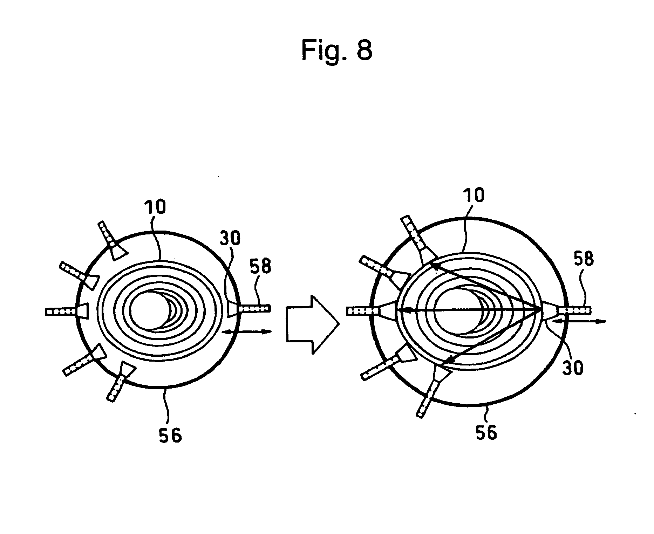

Instead of direct attaching to the outside of the tree by using a nail or clay as shown in Fig. 6, the sensors 30 may also be pressure-fitted to the surface of the tree by using a frame 56 and bolts 58 screwed in the frame as shown in Fig. 8.

After installing the sensors, the process advances to Step 120 of Fig. 5, and sensor position information is inputted into the measuring device 40. Next, a plurality of oscillators 32 are made to simultaneously multiple-oscillate at Step 130, and received signals are received by the small microphones 34. Herein, for identification of signals simultaneously multiple-oscillated, for example, the technique of Japanese Patent Application No. 2002-331057 proposed by a part of the applicant can be used.

At this Step 130, the speeds, energies, and frequency characteristics are measured. Herein, the speed is in proportion to the hardness of the tree and becomes low due to a hollow or decay, and is in inverse proportion to the moisture content. The energy becomes small due to a hollow or decay, and is in inverse proportion to the moisture content. The frequency characteristics are influenced by the size of a hollow or decay, and a hollow with a size of 1/4 or less of the wavelength cannot be grasped. Therefore, by performing measurement while changing the frequency, the size of a hollow can also be detected.

By comprehensively analyzing the sound speeds, energies, and frequency characteristics, results of analysis which can be interpreted more easily and whose accuracy is higher than convention can be obtained. On the other hand, conventionally, the information content is only one such as the speed in the case of using sound or the resistance in the case of using electricity, so that it is unknown which of hollow, decay, moisture content, and hardness influences the information, and as a result, it is difficult to interpret the result of measurement.

In addition, by measuring the speeds and frequency characteristics of energies and applying the Biot theory described in Japanese Published Unexamined Patent Applications No. 2000-35483, it becomes possible to grasp the water permeability of the inside of the tree. Normally, a healthy tree has high water permeability and sufficiently takes water, and on the other hand, a tree being dry inside or being sick is low in water permeability and water hardly flows inside.

After Step 130 is finished, waveform out putting and data reading are performed at Step 140. Then, at Step 150, the speed, attenuation rate, and internal information are obtained. Then, at Step 160, the results of detection of the tree section or the like are displayed.

The processing at Step 140 is performed as shown in Fig. 5 (B) indetail. Namely, atStep200, signals fromapluralityofoscillators are separated by multiple correlation calculation and are analyzed. Then, at Step 210, sound arrival times and amplitudes are read. Next, at Step 220, speed distribution and attenuation rate distribution are calculated from the arrival times and the amplitudes. Then, high-accuracy analysis is performed by means of inverse calculation at Step 230 if necessary.

Next, at Step 240, for example, a healthy area and a diseaseful and risky area are classified by colors and an image showing an internal state of the tree is outputted. Then, at Step 250, a level of risk is predicted by using a tree database.

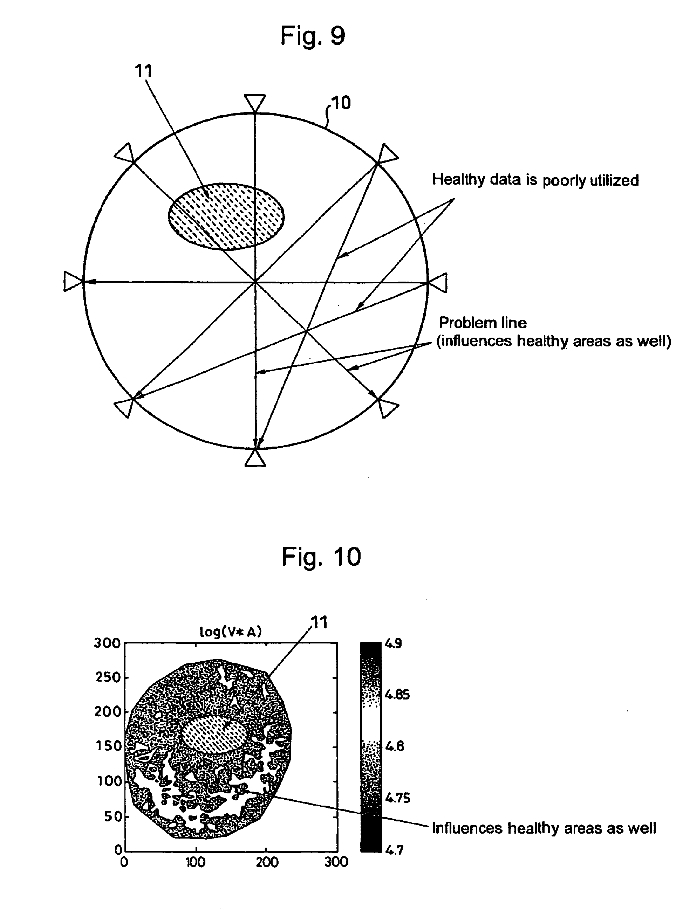

When drawing at Step 240, as shown in Fig. 9, if data on the measured lines are used without change, data passing through the hollow (decay) 11 influences as a problem line on a healthy area as shown in Fig. 10.

Therefore, the tree to be investigated is virtually divided and subjected to statistical processing, and then drawing is performed by using, for example, maximum values and average values in the divided areas. Thereby, healthy data of the healthy area is enhanced and displayed as healthy data when drawing.

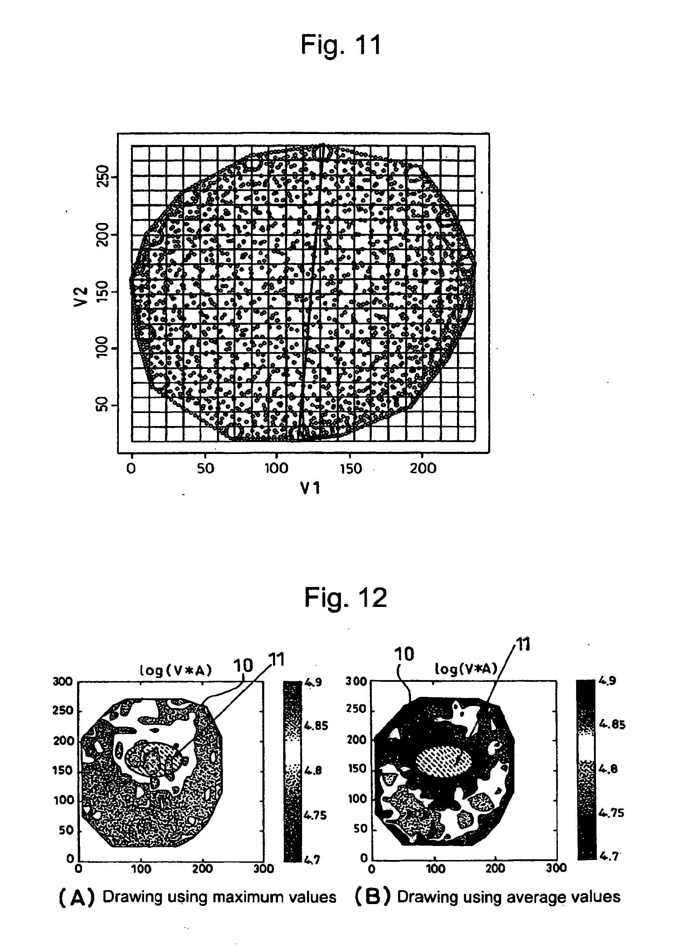

Fig. 11 is a diagram showing the process of collecting maximum and average value data, and first, collected data between survey points are plotted as points between the survey points. In this case, it is possible to designate the number of divisions between the survey points, and the number of divisions is, for example, 20.

Next, the tree is divided into grid areas of 20x20. After applying statistical processing, last, for example, a maximum value and an average value of points in a grid are extracted and set as representative values of the grid.

By using the representative values, maximum values and average values are drawn. An example is shown in Fig. 12.

In this embodiment, no problem line remains and healthy areas are displayed as healthy. For example, the maximum values are low in sensitivity and cannot express a small hole, however, it clearly express a large hole. On the other hand, average values are high in sensitivity and can express a small hole, however, they get blurred as a whole in expression of a large hole.

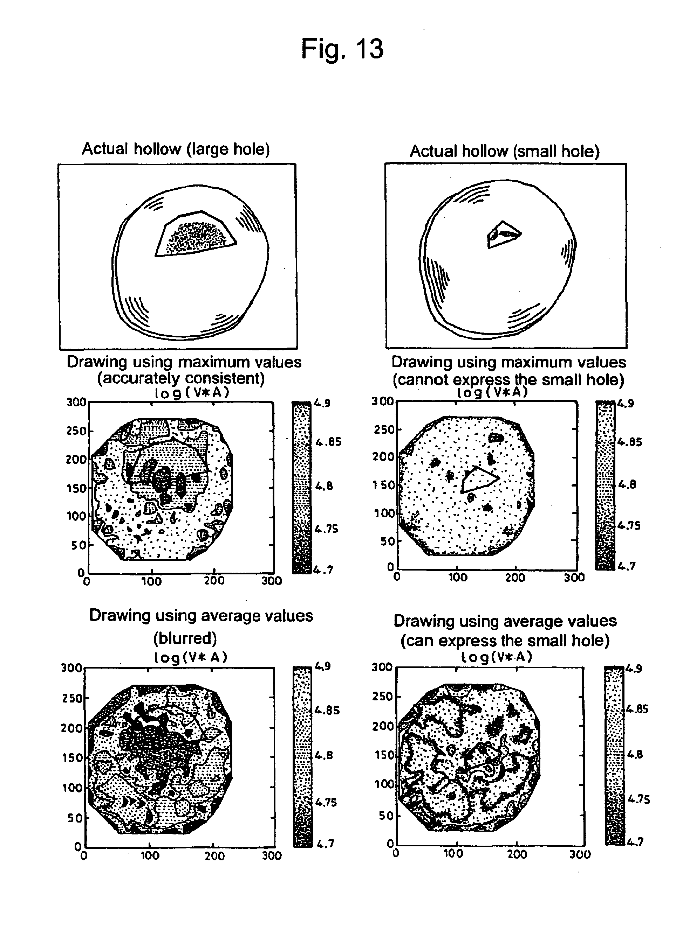

An example of the results of measurement according to the first embodiment is shown in Fig. 13. It is clearly seen that the results of measurement are accurately consistent with an actual hollow.

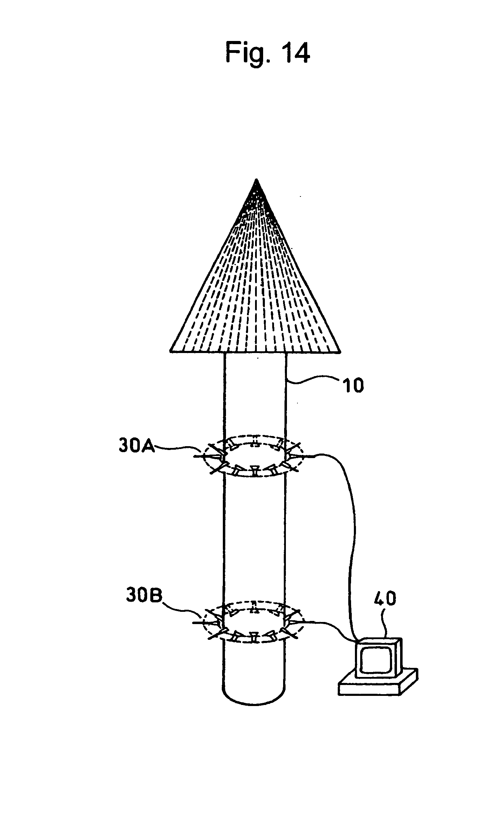

Next, a second embodiment of the invention will be described with reference to Fig. 14.

In this embodiment, two sensors 30A and 30B are located on upper and lower sides of the tree 10.

According to this embodiment, a state at a middle position between the two sensors 30A and 30B can also be inspected.

In the above embodiments, acoustic waves are converted into PRBS signals, so that high-accuracy measurement over a long distance can be made by using continuous waves. Depending on the purpose of use, conversion into the PRBS signals may be omitted.

In the above embodiments, a number of oscillators 32 and microphone 34 are disposed to simultaneously multiple-oscillate, so that the measurement time can be shortened. For example, it is also possible to repeatedly measure the tree while changing the positions of the pair of oscillator 32 and small microphone 34.

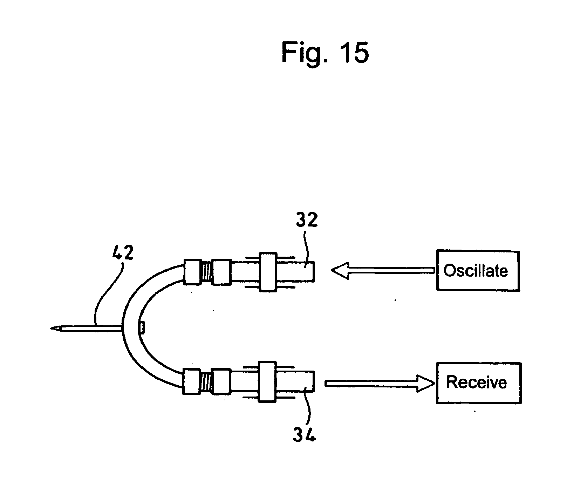

In the above embodiments, a piezoelectric device is used as the sensor, so that the sensor commonly serves as the oscillator and the microphone. As shown in Fig. 15, the oscillator 32 and the microphone 34 may be made separate from each other.

### [Industrial Applicability]

According to the invention, hollows and decay inside a roadside tree or an important tree can be diagnosed while it remains standing.

## Claims

1. An internal tree nondestructive inspection method using acoustic tomography, for inspecting an internal state of a tree (10) in a nondestructive manner, comprising the steps of:
making high-frequency acoustic waves generated by piezoelectric oscillators (32) whose amplitudes and frequencies are controlled so as to detect size of a hollow or decay (11) incident on the inside of the tree (10) from a plurality of positions;
receiving acoustic waves that are propagated inside the tree (10) at a plurality of positions; and
visualizing the internal state of the tree (10) based on the received signals
**characterized in that**
the plurality of piezoelectric oscillators (32) generate acoustic waves simultaneously, and a plurality of microphones (34) receive the simultaneously generated multiple-oscillate signals and identify each of the simultaneously generated multiple-oscillate signals and the acoustic waves are converted into pseudo random code binary sequence signals and that the simultaneously multiple-oscillate signals are divided and analyzed by multiple correlating calculation and statistically processed by inverse calculation.

2. The internal tree nondestructive inspection method using acoustic tomography according to Claim 1, wherein the hardness, existence of hollows and decay, and moisture content of the tree (10) are diagnosed from the speeds of the received signals.

3. The internal tree nondestructive inspection method using acoustic tomography according to Claim 1 or 2, wherein the existence of hollows and decay and the moisture content are diagnosed from energies of the received signals.

4. The internal tree nondestructive inspection method using acoustic tomography according to any of Claims 1 through 3, wherein the size of a hollow or decay is diagnosed from frequency characteristics of the received signals.

5. The internal tree nondestructive inspection method using acoustic tomography according to Claim 1, wherein the speeds, energies, and frequency characteristics of the received signals are comprehensively analyzed.

6. The internal tree nondestructive inspection method using acoustic tomography according to Claim 1, wherein water permeability is diagnosed from the speeds and frequency characteristics of energies of the received signals.

7. The internal tree nondestructive inspection method using acoustic tomography according to any of Claims 1 through 6, wherein when visualization is performed based on the received signals, a section of the tree (10) is virtually divided and drawing is performed by using statistical processing.

8. An internal tree nondestructive inspection apparatus using acoustic tomography for inspecting an internal state of a tree (10) in a nondestructive manner, comprising:
piezoelectric oscillators (32) for making high-frequency acoustic waves whose amplitudes and frequencies are controlled so as to detect size of a hollow or decay (11) incident on the inside of the tree (10);
microphones (34) for receiving the acoustic waves that are propagated inside the tree (10) at a plurality of positions; and
a computer for visualizing the internal state of the tree (10) based on the received signals.
**characterized in that**
the piezoelectric oscillators (32) are disposed generating acoustic waves simultaneously, and the microphones (34) are disposed receiving the simultaneously generated multiple-oscillate signals and identifying each of the simultaneously generated multiple-oscillate signals;
and that the acoustic waves are pseudo random code binary sequence signals and that the simultaneously multiple-oscillate signals are divided and analyzed by multiple correlating calculation and statistically processed by inverse calculation.

9. The internal tree nondestructive inspection apparatus using acoustic tomography according to Claim 8, wherein the piezoelectric oscillators (32) and the microphone (34) are formed to be common so that oscillating positions and receiving positions can be changed without changing the attachment positions to the tree (10).

## Patentansprüche

1. Verfahren zum zerstörungsfreien Prüfen eines Bauminneren mit Hilfe akustischer Tomografie für die Prüfung des inneren Zustandes eines Baumes (10) auf zerstörungsfreie Art und Weise, umfassend folgende Schritte:
Bewirken, dass Hochfrequenzschallwellen, die von piezoelektrischen Schwingkreisen (32) erzeugt werden, deren Amplituden und Frequenzen derart gesteuert werden, dass sie die Größe eines Hohlraumes oder eines Zerfalls (11) erfassen, auf das Innere des Baumes (10) aus einer Vielzahl von Positionen treffen;
Empfangen von Schallwellen, die sich im Inneren des Baumes (10) ausbreiten, an einer Vielzahl von Positionen; und
Visualisieren des inneren Zustandes des Baumes (10) auf der Basis der empfangenen Signale,
**dadurch gekennzeichnet, dass**
die Vielzahl der piezoelektrischen Schwingkreise (32) Schallwellen gleichzeitig erzeugt und eine Vielzahl von Mikrofonen (34) die gleichzeitig erzeugten Mehrfach-Schwingungssignale empfängt und jedes der gleichzeitig erzeugten Mehrfach-Schwingungssignale erkennt und die Schallwellen in Pseudozufallscode-Binärsequenzsignale umgewandelt werden, und dadurch, dass die gleichzeitigen Mehrfach-Schwingungssignale durch mehrfach korrelierende Berechnung unterteilt und analysiert sowie durch inverse Berechnung statistisch verarbeitet werden.

2. Verfahren zum zerstörungsfreien Prüfen eines Bauminneren mit Hilfe akustischer Tomografie nach Anspruch 1, bei dem Härte, Existenz von Hohlräumen und Zerfall sowie Feuchtegehalt des Baumes (10) anhand der Geschwindigkeiten der empfangenen Signale diagnostiziert werden.

3. Verfahren zum zerstörungsfreien Prüfen eines Bauminneren mit Hilfe akustischer Tomografie nach Anspruch 1 oder 2, bei dem die Existenz von Hohlräumen und Zerfall sowie der Feuchtegehalt anhand von Energien der empfangenen Signale diagnostiziert werden.

4. Verfahren zum zerstörungsfreien Prüfen eines Bauminneren mit Hilfe akustischer Tomografie nach einem der Ansprüche 1 bis 3, bei dem die Größe eines Hohlraumes oder Zerfalls anhand von Frequenzeigenschaften der empfangenen Signale diagnostiziert wird.

5. Verfahren zum zerstörungsfreien Prüfen eines Bauminneren mit Hilfe akustischer Tomografie nach Anspruch 1, bei dem die Geschwindigkeiten, die Energien und die Frequenzeigenschaften der empfangenen Signale umfassend analysiert werden.

6. Verfahren zum zerstörungsfreien Prüfen eines Bauminneren mit Hilfe akustischer Tomografie nach Anspruch 1, bei dem die Wasserdurchlässigkeit anhand der Geschwindigkeiten und Frequenzeigenschaften von Energien der empfangenen Signale diagnostiziert wird.

7. Verfahren zum zerstörungsfreien Prüfen eines Bauminneren mit Hilfe akustischer Tomografie nach einem der Ansprüche 1 bis 6, bei dem, wenn die Visualisierung auf der Basis der empfangenen Signale ausgeführt wird, ein Abschnitt des Baumes (10) virtuell unterteilt und eine Zeichnung mit Hilfe statistischer Verarbeitung erstellt wird.

8. Gerät zum zerstörungsfreien Prüfen eines Bauminneren mit Hilfe akustischer Tomografie für die Prüfung des inneren Zustandes eines Baumes (10) auf zerstörungsfreie Art und Weise, umfassend:
piezoelektrische Schwingkreise (32), die bewirken, dass Hochfrequenzschallwellen, deren Amplituden und Frequenzen derart gesteuert werden, dass die Größe eines Hohlraumes oder Verfalls (11) erfasst wird, auf das Innere des Baumes (10) treffen;
Mikrofone (34) zum Empfangen der Schallwellen, die sich im Inneren des Baumes (10) ausbreiten, an einer Vielzahl von Positionen; und
einen Computer zum Visualisieren des inneren Zustandes des Baumes (10) auf der Basis der empfangenen Signale,
**dadurch gekennzeichnet, dass**
die piezoelektrischen Schwingkreise (32) dazu eingerichtet sind, Schallwellen gleichzeitig zu erzeugen, und die Mikrofone (34) dazu eingerichtet sind, die gleichzeitig erzeugten Mehrfach-Schwingungssignale zu empfangen und jedes der gleichzeitig erzeugten Mehrfach-Schwingungssignale zu erkennen;
und dass die Schallwellen Pseudozufallscode-Binärsequenzsignale sind und die gleichzeitigen Mehrfach-Schwingungssignale durch mehrfach korrelierende Berechnung unterteilt und analysiert sowie durch inverse Berechnung statistisch verarbeitet werden.

9. Gerät zum zerstörungsfreien Prüfen eines Bauminneren mit Hilfe akustischer Tomografie nach Anspruch 8, bei dem die piezoelektrischen Schwingkreise (32) und die Mikrofone (34) herkömmlich ausgebildet sind, so dass Schwingungspositionen und Empfangspositionen geändert werden können, ohne die Anbringungspositionen an dem Baum (10) zu ändern.

## Revendications

1. Procédé d'inspection non destructif de l'intérieur d'un arbre utilisant une tomographie acoustique, pour inspecter un état interne d'un arbre (10) d'une manière non destructive, comprenant les étapes consistant à :
créer des ondes acoustiques haute fréquence générées par des oscillateurs piézoélectriques (32) dont les amplitudes et les fréquences sont commandées de manière à détecter une taille d'un incident à type de cavité ou de pourriture (11) à l'intérieur de l'arbre (10) à partir d'une pluralité de positions ;
recevoir des ondes acoustiques propagées à l'intérieur de l'arbre (10) au niveau d'une pluralité de positions ; et
visualiser l'état interne de l'arbre (10) sur la base des signaux reçus
**caractérisé en ce que**
la pluralité d'oscillateurs piézoélectriques (32) génèrent des ondes acoustiques de manière simultanée, et une pluralité de microphones (34) reçoivent les signaux à oscillations multiples générés de manière simultanée et identifient chacun des signaux à oscillations multiples générés de manière simultanée et les ondes acoustiques sont converties en signaux à séquence binaire pseudo-aléatoire et les signaux à oscillations multiples simultanés sont divisés et analysés par un calcul de corrélation multiple et traités de manière statistique par calcul inverse.

2. Procédé d'inspection non destructif de l'intérieur d'un arbre utilisant une tomographie acoustique selon la revendication 1, dans lequel la dureté, l'existence de cavités et de pourriture, et une teneur en humidité de l'arbre (10) sont diagnostiquées à partir des vitesses des signaux reçus.

3. Procédé d'inspection non destructif de l'intérieur d'un arbre utilisant une tomographie acoustique selon la revendication 1 ou 2, dans lequel l'existence de cavités et de pourriture et la teneur en humidité sont diagnostiquées à partir des énergies des signaux reçus.

4. Procédé d'inspection non destructif de l'intérieur d'un arbre utilisant une tomographie acoustique selon l'une quelconque des revendications 1 à 3, dans lequel la taille d'une cavité ou d'une pourriture est diagnostiquée à partir des caractéristiques de fréquence des signaux reçus.

5. Procédé d'inspection non destructif de l'intérieur d'un arbre utilisant une tomographie acoustique selon la revendication 1, dans lequel les vitesses, énergies et caractéristiques de fréquence des signaux reçus sont analysées de manière détaillée.

6. Procédé d'inspection non destructif de l'intérieur d'un arbre utilisant une tomographie acoustique selon la revendication 1, dans lequel une perméabilité à l'eau est diagnostiquée à partir des vitesses et des caractéristiques de fréquence des énergies des signaux reçus.

7. Procédé d'inspection non destructif de l'intérieur d'un arbre utilisant une tomographie acoustique selon l'une quelconque des revendications 1 à 6, dans lequel, lorsqu'une visualisation est réalisée sur la base des signaux reçus, une section de l'arbre (10) est divisée de manière virtuelle et une représentation dessinée est réalisée en utilisant un traitement statistique.

8. Appareil d'inspection non destructif de l'intérieur d'un arbre utilisant une tomographie acoustique pour inspecter un état interne d'un arbre (10) d'une manière non destructive, comprenant :
des oscillateurs piézoélectriques (32) pour créer des ondes acoustiques haute fréquence dont les amplitudes et les fréquences sont commandées de manière à détecter une taille d'un incident à type de cavité ou de pourriture (11) à l'intérieur de l'arbre (10) ;
des microphones (34) pour recevoir les ondes acoustiques propagées à l'intérieur de l'arbre (10) au niveau d'une pluralité de positions ; et
un ordinateur pour visualiser l'état interne de l'arbre (10) sur la base des signaux reçus,
**caractérisé en ce que**
les oscillateurs piézoélectriques (32) sont disposés de manière à générer des ondes acoustiques de manière simultanée, et les microphones (34) sont disposés de manière à recevoir des signaux à oscillations multiples générés de manière simultanée et à identifier chacun des signaux à oscillations multiples générés de manière simultanée ;
et les ondes acoustiques sont des signaux à séquence binaire pseudo-aléatoire et les signaux à oscillations multiples simultanés sont divisés et analysés par un calcul de corrélation multiple et traités de manière statistique par calcul inverse.

9. Appareil d'inspection non destructif de l'intérieur d'un arbre utilisant une tomographie acoustique selon la revendication 8, dans lequel les oscillateurs piézoélectriques (32) et les microphones (34) sont formés communs de manière à ce que des positions d'oscillation et des positions de réception puissent être modifiées sans modifier les positions de fixation sur l'arbre (10).
